# EUROPÄISCHE PATENTSCHRIFT

(11) **EP 3 228 292 B1**
(45) Veröffentlichungstag und Bekanntmachung des Hinweises auf die Patenterteilung: **30.09.2020**
(21) Anmeldenummer: 16164554.4
(22) Anmeldetag: 08.04.2016
(51) Int. Cl.: A61F 13/49, B32B 5/04, B32B 7/14, B32B 27/12, C08J 5/18, C09J 153/02, C08L 53/02

(54) **ELASTISCHES LAMINAT SOWIE VERFAHREN ZUR HERSTELLUNG DES ELASTISCHEN LAMINATES**
ELASTIC LAMINATE AND PROCESS FOR PRODUCING THE ELASTIC LAMINATE
LAMINE ELASTIQUE ET PROCEDE DE PRODUCTION DU LAMINE ELASTIQUE

(43) Veröffentlichungstag der Anmeldung: 11.10.2017
(73) Patentinhaber: Mondi AG, 1030 Wien (AT)
(72) Erfinder: Baldauf, Georg, 48366 Laer (DE); Schönbeck, Marcus, 33775 Versmold (DE); Sollmann, Henner, 48599 Gronau (DE)
(74) Vertreter: Lorenz, Bernd Ingo Thaddeus

(56) Entgegenhaltungen:
- EP-A1- 1 686 209
- EP-A1- 2 799 228
- EP-A2- 1 541 106
- WO-A1-2015/037507
- US-A1- 2014 073 211

## Beschreibung

Die Erfindung betrifft ein elastisches Laminat für Windelverschlüsse, ein Verfahren zur Herstellung eines elastischen Laminates sowie eine mit dem elastischen Laminat gebildete Einweg-Windel.

Windeln, insbesondere für Babys und Kleinkinder werden als Einweg-Massenartikel produziert, so dass sich besondere Anforderungen an eine effiziente Materialausnutzung sowie geringe Gestehungskosten ergeben.

Darüber hinaus ist jedoch gleichzeitig auch ein großes Maß an Funktionalität notwendig, damit unter allen Einsatzbedingungen ein hohes Maß an Sicherheit sowie ein zuverlässiger Schutz der die Windel tragenden Person sichergestellt werden. Über Nacht müssen große Mengen an Exkrementen aufgenommen und sicher zurückgehalten werden, wobei möglichst auch Beeinträchtigungen der Haut eines Trägers vermieden werden sollen. Zur Abdichtung an der Taille sowie den Beinen eines Benutzers sind deshalb Einweg-Windeln mit elastischen Elementen und Abschnitten versehen. Gleichzeitig muss aber auch bei einer Bewegung des Benutzers ein hoher Tragekomfort sichergestellt werden. Insbesondere sind Einschnürungen und Hautirritationen aufgrund der elastischen Abschnitte und Elemente zu vermeiden.

Um elastische Seitenteile und insbesondere seitliche Windelverschlüsse zu bilden, sind elastische Laminate bekannt, welche an ihren gegenüberliegenden Außenseiten Lagen aus Nonwoven und dazwischen einen elastischen Kern aufweisen. Der elastische Kern kann gemäß dem Stand der Technik beispielsweise durch eine vollflächig eingebundene elastische Folie oder auch elastische Stränge gebildet werden.

Ein gattungsgemäßes elastisches Laminat für Windelverschlüsse mit zwei gegenüberliegende Außenseiten bildenden Nonwoven-Lagen und mit in einer Dehnungsrichtung verlaufenden, zwischen den Nonwoven-Lagen durch Klebstoff eingebundenen Streifen einer elastischen Folie ist aus der EP 1 928 380 B1 bekannt. Eine elastische Schicht ohne Deckschichten kann entweder vollflächig oder in Form von Streifen zwischen den Nonwoven-Lagen eingebunden werden, wobei das so gebildete Material zunächst aufgrund der Nonwoven-Lagen nur mit einem erheblichen Kraftaufwand dehnbar ist und deshalb zunächst einer Aktivierung unterzogen wird. Bei der Aktivierung durch Ringrollen, die beispielsweise aus US 4 834 741 bekannt sind, wird die Struktur der außenliegenden Nonwoven-Lagen gedehnt und in der Regel auch teilweise zerstört. Zumindest bis zu der durch die Aktivierung vorgegebenen Dehngrenze ist das aktivierte elastische Laminat nachfolgend bei seiner Benutzung leicht dehnbar. Für die Aktivierung ist dabei ein weiterer Verfahrensschritt notwendig, wobei je nach Material der Nonwoven-Lagen bei der Aktivierung auch eine strukturelle Schädigung nicht ausgeschlossen werden kann, welche bei einer Benutzung des elastischen Laminates zu einer versehentlichen Überdehnung oder auch zu einem Zerreißen führen kann, weil je nach konkreter Ausgestaltung die überdehnten Nonwoven-Lagen nur zu einem geringen Anteil zu der Stabilität des elastischen Laminates beitragen können. Vielmehr wird die notwendige Stabilität des elastischen Laminates von den zwischen den Nonwoven eingebundenen Streifen bereitgestellt, so dass diese eine ausreichende Zugfestigkeit aufweisen müssen.

Aus dem Stand der Technik ist des Weiteren bekannt, vollflächige Folien oder einzelne elastische Filamente in einem gedehnten Zustand zwischen zwei Nonwoven-Lagen einzubinden, wobei dann die Nonwoven-Lagen bei einem Wegfall der Zugkräfte in Falten gelegt werden, so dass sich bei der Verwendung eines solchen Materials für eine Windel ein besonders weicher und angenehmer Sitz ergibt. Des Weiteren ergibt sich der Vorteil, dass der Grad der Vorspannung der eingebundenen elastischen Elemente eine klare Dehngrenze vorgibt. Das elastische Laminat kann so weit gedehnt werden, wie es sich zuvor nach Wegfall der Zugkräfte kontrahiert hat. Die Struktur der Nonwoven-Lagen wird dabei nicht wesentlich verändert. Insbesondere weisen die Nonwoven-Lagen auch bei Erreichen der durch die Vorspannung vorgegebenen Dehnungsgrenze eine hohe Stabilität auf und sind in ihrer Struktur nicht durch eine Aktivierung vorgeschädigt.

Die Einbindung einzelner Filamente in einem gedehnten Zustand ist beispielsweise aus EP 1 179 330 B1, EP 1 610 950 B1, EP 1 707 351 A1 sowie EP 1 425 171 B1 bekannt. Die Handhabung der einzelnen Filamente ist während der Herstellung aufwändig.

Der vorliegenden Erfindung liegt die Aufgabe zugrunde, ein elastisches Laminat sowie ein Verfahren zu dessen Herstellung anzugeben, wobei das elastische Laminat sich durch besonders vorteilhafte und weiche mechanische Eigenschaften bei geringen Herstellungskosten auszeichnen soll. Des Weiteren soll eine mit dem elastischen Laminat gebildete Einweg-Windel angegeben werden.

Gegenstand der Erfindung und Lösung der Aufgabe sind ein elastisches Laminat gemäß Patentanspruch 1, ein Verfahren zur Herstellung eines elastischen Laminates gemäß Patentanspruch 6 sowie eine Einweg-Windel gemäß Patentanspruch 10.

Ausgehend von einem elastischen Laminat mit den eingangs beschriebenen Merkmalen ist demnach erfindungsgemäß vorgesehen, dass die Streifen unter Vorspannung zwischen den Nonwoven-Lagen eingebunden sind und die Nonwoven-Lagen in einem entspannten Zustand durch die Rückstellkräfte der unter Vorspannung eingebundenen Streifen entlang der Dehnungsrichtung in Falten gelegt, also gerafft sind, wobei in dem entspannten Zustand die Dicke der Streifen zwischen 30 µm und 80 µm und die senkrecht zu der Dehnungsrichtung bestimmte Breite der Streifen zwischen 3 mm und 12 mm beträgt und wobei die Streifen der elastischen Folie im entspannten Zustand zwischen 10 % und 80 % der Gesamtfläche, also der Gesamtfläche des Laminates, abdecken.

Typischerweise verlaufen die Streifen mit einem gleichen Abstand und mit geraden Rändern parallel zueinander. Vorzugsweise sind die Streifen unmittelbar, d. h. ohne weitere Zwischenlagen mit den Nonwoven-Lagen verklebt.

Durch die Einbindung der Streifen der elastischen Folie in einem gedehnten Zustand wird eine klar definierte Dehngrenze vorgegeben. Nach Wegfall der Zugkräfte nach der Herstellung des Laminates stellt sich dieses so weit zurück, bis die Streifen der elastischen Folie wieder ihre Ausgangslänge im kräftefreien Zustand erreicht haben.

Gemäß der entsprechenden Vorspannung der Streifen der elastischen Folie kann das elastische Laminat beispielsweise bis zu einer Dehngrenze mit einer Dehnung zwischen 30 % und 300 %, insbesondere zwischen 50 % und 250 % dehnbar sein. Im Rahmen der Erfindung ergibt sich der Vorteil, dass die Stabilität und Zugfestigkeit des elastischen Laminates bei Erreichen der Dehngrenze zu wesentlichen Teilen von den Nonwoven-Lagen bestimmt wird. Auch wenn die Streifen selbst besonders leicht dehnbar sind und somit das elastische Laminat mit geringem Kraftaufwand bis zu der Dehngrenze gedehnt werden kann, können die in ihrer Struktur nicht durch eine gemäß dem Stand der Technik übliche Aktivierung geschädigten Nonwoven-Lagen eine hohe Festigkeit bereitstellen. Daraus ergibt sich, dass im Gegensatz zu einem aktivierten elastischen Laminat besonders leicht dehnbare elastische Folien mit einer vergleichsweise geringen Zugfestigkeit eingesetzt werden können.

Die elastische Folie weist in dem entspannten Zustand eine Dicke von lediglich 30 µm bis 80 µm auf, wobei die Breite der daraus gebildeten Streifen zwischen 3 mm und 12 mm beträgt. Die Streifen der elastischen Folie liegen damit flach zwischen den beiden Nonwoven-Lagen und tragen folglich hinsichtlich der Dicke nicht wesentlich auf. Durch die flache Anordnung der Streifen können auch die elastischen Eigenschaften des Materials optimal ausgenutzt werden, wobei durch die Flächenbelegung zwischen 10 % und 80 % nicht nur gleichmäßige elastische Eigenschaften, sondern auch eine vorteilhafte Faltenstruktur der Nonwoven-Lagen zwischen den elastischen Streifen im entspannten Zustand erreicht wird.

Die Anordnung der Nonwoven-Lagen im entspannten Zustand kann auch als Rüscheneffekt bezeichnet werden, wobei sich eine besonders weiche und angenehme Oberfläche ergibt, welche auch bei einem direkten Hautkontakt einen Benutzer optimal schont.

Gemäß einer bevorzugten Weiterbildung der Erfindung sind die Nonwoven-Lagen mit den Streifen und zwischen den Streifen miteinander vollflächig verklebt. Durch einen vollflächigen Auftrag von Klebstoff wird eine gute Verbundhaftung des elastischen Laminats erreicht, wobei sich auch eine einfache Verfahrensführung ergibt. Der vollflächige Auftrag von Klebstoff kann beispielsweise durch Sprühdüsen oder eine Walze erfolgen, wobei auf die aufwändige Erzeugung eines Musters verzichtet werden kann.

Als Klebstoff ist gemäß einer bevorzugten Weiterbildung ein Hotmelt-Klebstoff vorgesehen. Dieser Hotmelt-Klebstoff weist zweckmäßigerweise elastische Eigenschaften auf, damit die Rückstellung der unter Vorspannung zwischen den Nonwoven-Lagen eingebundenen Streifen nicht behindert wird. Bevorzugt kann ein Hotmelt-Klebstoff auf der Basis von Styrol-Block-Copolymer, beispielsweise Styrol-Isopren-Styrol-Block-Copolymer (SIS) oder StyrolButadien-Styrol-Copolymer (SBS) eingesetzt werden. Des Weiteren kommen auch Hotmelt-Klebstoffe auf der Basis von Polyolefin-Elastomer in Betracht.

Die Streifen der elastischen Folie decken im entspannten Zustand bevorzugt zwischen 30 % und 65 % der Gesamtfläche ab. Der senkrecht zu der Dehnungsrichtung im entspannten Zustand bestimmte Abstand benachbarter Streifen beträgt gemessen von Streifenrand zu Streifenrand zweckmäßigerweise zwischen 3 mm und 12 mm. Somit ergibt sich beispielsweise eine Abdeckung von 50 %, wenn die Breite der Streifen auch dem Abstand benachbarter Streifen entspricht.

Gegenstand der Erfindung ist auch ein Verfahren zur Herstellung eines elastischen Laminates, insbesondere des zuvor beschriebenen Laminates. Das Verfahren umfasst die Schritte
Extrudieren einer elastischen Folie mit einer Dicke zwischen 30 µm und 80 µm,
Zerschneiden der elastischen Folie zur Bildung von Streifen,
Einbringen der elastischen Streifen in einem gedehnten Zustand mit einer Dehnung zwischen 30 % und 300 %, insbesondere zwischen 50 % und 250 %, sowie mit einem Abstand zueinander zwischen zwei Nonwoven-Lagen,
Kaschieren der Nonwoven-Lagen mit den Streifen im gedehnten Zustand mittels Klebstoff,
Entspannen des aus den Nonwoven-Lagen und den Streifen gebildeten Laminates.

Die Produktionsrichtung in der die elastische Folie als Monofolie extrudiert oder vorzugsweise als mehrschichtige Folie koextrudiert wird, wird auch als Maschinenrichtung bezeichnet. Vorzugsweise wird die Folie so in Streifen geschnitten, dass auch die einzelnen Streifen entlang der Maschinenrichtung verlaufen, was alleine hinsichtlich der Verfahrensführung von Vorteil ist, weil dann auch die Streifen als Endlosmaterial gebildet und leicht weiterverarbeitet werden können.

Gerade bei einem erstmaligen Dehnen der Zwischenfolie ist häufig eine deutlich größere Kraft für eine Dehnung um einen Folienwert notwendig, als bei darauf folgenden Dehnungen. Dies trifft insbesondere dann zu, wenn wie nachfolgend noch im Detail beschrieben, die elastische Folie mit dünnen nicht-elastischen Deckschichten versehen ist, so dass für die Bereitstellung einer leichten Dehnbarkeit die elastische Folie selbst zunächst in einem gewissen Maße aktiviert werden muss. Vor diesem Hintergrund ist es gemäß einer bevorzugen Ausgestaltung des Verfahrens vorgesehen, dass entweder die extrudierte Folie oder die daraus geschnittenen Streifen vor dem Einbringen zwischen die Nonwoven-Lagen zunächst vorgedehnt werden. Insbesondere ist diese erste Vordehnung vorzugsweise stärker als die Vorspannung, mit der die elastischen Streifen im gedehnten Zustand mit den Nonwoven-Lagen kaschiert werden. Durch die stärkere Vordehnung wird sichergestellt, dass zumindest bis zu der durch das Verfahren vorgegebenen Dehngrenze eine besonders leichte Dehnung der elastischen Folie bzw. der daraus gebildeten Streifen möglich ist.

So kann beispielsweise vorgesehen sein, dass die extrudierte elastische Folie oder die daraus geschnittenen Streifen vor dem Einbringen zwischen die Nonwoven-Lagen um 300 % bis 500 % vorgedehnt werden, wobei die dann nach einer Spannung resultierende Länge mit einer permanenten Verformung (permanent set) als Ausgangslänge für die nachfolgende Dehnung während der Kaschierung zugrunde zu legen ist.

Um eine einfache Verfahrensführung zu ermöglichen werden gemäß einer bevorzugten Weiterbildung der Erfindung, die beiden Nonwoven-Lagen vor dem Kaschieren mit Hotmelt-Klebstoff, insbesondere vollflächig mit Hotmelt-Klebstoff beschichtet.

Ein weiterer Aspekt der vorliegenden Erfindung betrifft die konkrete Ausgestaltung der elastischen Folie. Die elastische Folie weist gemäß dem weiteren Aspekt der vorliegenden Erfindung eine elastische Folienschicht auf der Basis von Styrol-Block-Copolymer auf und ist dadurch gekennzeichnet, dass die elastische Folienschicht ohne die Zugabe von Polystyrol von einem ersten Styrol-Block-Copolymer und einem zweiten Styrol-Block-Copolymer gebildet ist, wobei die beiden Styrol-Block-Copolymere einen unterschiedlichen Gewichtsanteil an Styrol sowie vorzugsweise auch eine unterschiedliche Härte aufweisen. Durch die Kombination von zwei Styrol-Block-Copolymeren kann eine besonders leichte Dehnbarkeit bei einer gleichzeitig ausreichenden Festigkeit realisiert werden.

Konkret ist vorgesehen, dass das erste Styrol-Block-Copolymer einen StyrolAnteil von weniger als 25 Gew.%, vorzugsweise weniger als 22 Gew.-%, beispielsweise zwischen 10 und 20 Gew.-% und eine Härte von weniger als 45 Shore A aufweist, wobei das zweite Styrol-Block-Copolymer einen StyrolAnteil von mehr als 26 Gew.-%, insbesondere zwischen 30 und 40 Gew.-% sowie eine Härte von mehr als 45 Shore A aufweist. Das Verhältnis des ersten, weichen Styrol-Block-Copolymers zu dem zweiten Styrol-Block-Copolymer liegt davon ausgehend in einem Bereich zwischen 6:1 und 2:3, insbesondere zwischen 5:1 und 1:1.

Ohne die Zugabe von Polystyrol und aufgrund des ersten Anteils des ersten Styrol-Block-Copolymers von zumindest 40 % im Vergleich zu dem zweiten Styrol-Block-Copolymer weist die elastische Folienschicht eine leichte Dehnbarkeit, jedoch auch eine geringe Zugfestigkeit und hohe Klebrigkeit auf.

Durch die erfindungsgemäße Zusammenstellung der elastischen Folienschicht werden gerade auch in Maschinenrichtung gute elastische Eigenschaften erreicht, während gemäß dem Stand der Technik häufig die Dehnung der elastischen Folie in Querrichtung vorgesehen ist und dort entsprechend die Dehnungseigenschaften in Querrichtung im Vordergrund stehen.

Wie bereits zuvor erläutert, wird die elastische Folie bei der Erzeugung von Streifen allein aus praktischen Gründen üblicherweise entlang der Maschinenrichtung geschnitten, so dass diese Ausrichtung der Streifen auch der späteren Dehnungsrichtung des Laminates entspricht. Aufgrund dieser verfahrenstechnischen und strukturellen Vorgaben steht im Rahmen der Erfindung gerade die Dehnbarkeit der elastischen Folie in Maschinenrichtung also entlang der bei der Extrusion gerichteten Polymerstränge im Vordergrund. In diesem Zusammenhang wird angenommen, dass gerade durch die Kombination von zwei hinsichtlich ihrer mechanischen Eigenschaften unterschiedlichen Styrol-Block-Copolymeren und das Weglassen von Polystyrol als übliche Zugabe gerade in der Maschinenrichtung die notwendige Elastizität erreicht werden kann.

Gemäß einer bevorzugten Ausgestaltung der Erfindung ist vor diesem Hintergrund vorgesehen, dass die elastische Folienschicht bei einem durch Coextrusion gebildeten dreischichtigen Aufbau zwischen zwei nicht-elastischen Deckschichten angeordnet ist. Dieser dreischichtige Aufbau ist aus mehreren Gründen vorteilhaft. Zunächst kann die so gebildete elastische Folie ohne Weiteres aufgerollt, transportiert und wieder abgerollt werden, ohne aufgrund der Klebrigkeit der elastischen Folienschicht auf der Basis von Styrol-Block-Copolymeren im aufgerollten Zustand zu verblocken. Hinzukommt, dass die Deckschichten die elastische Folienschicht sowohl bei dem Coextrusionsprozess als auch bei der nachfolgenden Verarbeitung stabilisieren können. Bei einer nachfolgenden Verarbeitung kann die elastische Folie beispielsweise ohne eine übermäßige Dehnung auf- und abgerollt werden, solange die Deckschichten in ihrer Struktur nicht beschädigt werden.

Bei der Coextrusion können die nicht-elastischen Deckschichten auch eine Begrenzung und Stabilisierung des weichen Styrol-Block-Copolymers bewirken.

Gerade wenn die elastische Folie nicht-elastische Deckschichten aufweist, kann bei dem zuvor beschriebenen Verfahren eine Vordehnung vor dem Verbinden mit den Nonwoven-Lagen zweckmäßig sein, um die elastische Folie durch eine Überdehnung der Deckschichten zu aktivieren. Es ergibt sich dann auch der Vorteil, dass die Deckschichten nach einer solchen Aktivierung die elastischen Eigenschaften der elastischen Folie nicht wesentlich beeinträchtigen können.

Des Weiteren ist vorgesehen, dass die nicht-elastischen Deckschichten jeweils nur eine geringe Dicke zwischen beispielsweise 1,5 µm und 6 µm, insbesondere 2 µm und 4 µm aufweisen. Zunächst kann auch durch eine geringe Dicke sichergestellt werden, dass eine Vordehnung zur Aktivierung der elastischen Folie leicht möglich ist und dass die elastischen Eigenschaften der elastischen Folie nicht wesentlich durch die Deckschichten beeinträchtigt werden. Darüber hinaus sind die Deckschichten bei der Coextrusion und der Verarbeitung für eine Stabilisierung der elastischen Folienschicht auf der Basis von Styrol-Block-Copolymeren vorgesehen, so dass auch dazu eine geringe Dicke ausreichend ist. Schließlich ist der Fachmann bestrebt, aus Gründen der Materialeinsparung die einzelnen Schichten nur mit der für die jeweilige Funktion notwendigen Dicke auszuführen.

Die nicht-elastischen Deckschichten können beispielsweise aus Polyolefin, insbesondere aus Polyethylen, Polypropylen oder Mischungen von Polyethylen und Polypropylen gebildet sein. Entsprechend Polyolefine sind kostengünstig und in der Regel auch relativ leicht dehnbar.

Die Dicke der elastischen Folienschicht beträgt vorzugsweise zwischen 30 µm und 60 µm, insbesondere zwischen 40 µm und 50 µm.

Wie bereits zuvor erläutert, wird im Rahmen der Erfindung eine besonders gleichmäßige und leichte Dehnbarkeit eines unter Einsatzes der elastischen Folie gebildeten elastischen Laminates angestrebt. Wenn Streifen der elastischen Folie unter Vorspannung zwischen zwei Nonwoven-Lagen eingebunden werden, wird dadurch eine Dehngrenze des elastischen Laminates durch diese Vorspannung bestimmt. Dies führt auch dazu, dass die elastische Folie und insbesondere die elastische Folienschicht zwar gute elastische Rückstelleigenschaften aufweisen soll, jedoch insgesamt nur eine geringe Zugfestigkeit aufweisen muss. Bei Erreichen der durch die Vorspannung vorgegebenen Dehngrenze wird eine weitere Dehnung durch die außenliegenden Nonwoven-Lagen begrenzt, was üblicherweise durch einen rapiden Kraftanstieg bei einer weiteren Dehnung für den Benutzer unmittelbar wahrnehmbar ist.

Neben einer leichten Dehnbarkeit soll das elastische Laminat jedoch auch ausreichende Haltekräfte aufweisen. Dies kann dadurch sichergestellt werden, dass die elastische Folie sowie ein damit gebildetes elastisches Laminat weitgehend elastische Eigenschaften aufweisen und die bei der Dehnung aufgewandte Energie bei einer Kontraktion wieder zu einem großen Teil zurückgewonnen werden kann.

Hierzu definiert die EP 1 928 380 B1 einen Energierückgewinnungswert (energy recovery value) auf dessen Definition im Rahmen der vorliegenden Erfindung in vollem Umfang verwiesen wird. Die vorliegende Erfindung macht sich insbesondere das in diesem erteilten Patent definierte Testverfahren zu eigen.

Demnach kann ein MTS alliance RT/1-Testsystem zur Bestimmung von Zugkräften eingesetzt werden, um in einem Dehnungsversuch eine Hysterese zu bestimmen, auf dessen Basis der Energierückgewinnungswert (energy recovery value) bestimmt wird. Der Energierückgewinnungswert gibt den Prozentsatz an Energie an, die bei einer Dehnung und einer Entspannung eines Testmusters um 200 % wieder zurückgewonnen wird. Hierzu wird ein Testmuster in einer entsprechenden Versuchsanordnung angeordnet, wobei während der Dehnung der darauf folgenden Kontraktion bei einer vorgegebenen Testgeschwindigkeit gemäß der EP 1 928 380 B1 die wirkenden Kräfte aufgezeichnet werden. Es ergibt sich dabei in einem üblichen Weg-Kraft-Diagramm eine Hysterese, wobei sich der Energierückgewinnungswert als Quotient der Integrale der Kurven für die Entspannung einerseits und die Dehnung andererseits ergibt.

Für ein ideales elastisches Material ohne Hysterese ergibt sich damit ein theoretischer Energierückgewinnungswert von 1. Im Rahmen der Erfindung liegt der Energierückgewinnungswert für die elastische Folie, insbesondere in Maschinenrichtung vorzugsweise oberhalb von 0,6, insbesondere oberhalb von 0,68.

Wie bereits zuvor erläutert, kann für eine erstmalige Aktivierung der elastischen Folie eine Vordehnung vorgesehen sein.

Vor diesem Hintergrund ist im Rahmen der Erfindung vorzugsweise vorgesehen, dass nach einer ersten Aktivierung mit einer Dehnung von 500 % und einer Rückstellung der elastischen Folie die dann für eine Dehnung von 100 % notwendige Kraft weniger als 1 N pro 19 mm Probebreite beträgt. Insbesondere liegt die Kraft zwischen 0,5 und 1 N pro 19 mm Probebreite, wobei die elastische Folie die genannten Eigenschaften vorzugsweise in Maschinenrichtung aufweist.

Entsprechend beträgt nach einer ersten Aktivierung mit einer Dehnung von 500 % und einer Rückstellung der elastischen Folie die für eine weitere Dehnung von 200 % notwendige Kraft weniger als 1,5 N pro 19 mm und insbesondere zwischen 1 und 1,5 N pro 19 mm bezogen auf die Maschinenrichtung.

Als erstes und zweites Styrol-Block-Copolymer ist im Rahmen der Erfindung vorzugsweise ein Styrol-Isopren-Styrol-Block-Copolymer (SIS) vorgesehen.

Schließlich betrifft die Erfindung auch eine Einweg-Windel, welche mit dem zuvor beschriebenen elastischen Laminat für Windelverschlüsse gebildet ist. Die Einweg-Windel umfasst einen Windel-Grundkörper, der zwischen einer flüssigkeitsdichten Außenlage und einer flüssigkeitsdurchlässigen Innenlage einen Flüssigkeit aufnehmenden Kern aufweist, wobei zwei Abschnitte des zuvor beschriebenen elastischen Laminates an den Windelgrundkörper angeschlossen und als seitliche Verbindung zwischen einem vorderen Teil und einem hinteren Teil des Windel-Grundkörpers vorgesehen sind. Insbesondere können die Abschnitte jeweils an einem ersten Ende an dem Windel-Grundkörper angeschlossen sein und an einem zweiten Ende ein Verschlusselement, beispielsweise ein Hakenmaterial, tragen.

Die Erfindung wird im Folgenden anhand einer lediglich ein Ausführungsbeispiel darstellenden Zeichnung erläutert. Es zeigen:
- **Fig. 1**: ein elastisches Laminat für Windelverschlüsse in einem entspannten Zustand,
- **Fig. 2**: das elastische Laminat gemäß der Fig. 1 in einem vollständig gedehnten Zustand an einer Dehnungsgrenze,
- **Fig. 3**: einen Querschnitt durch das elastische Laminat,
- **Fig. 4a**: Weg-Kraft-Diagramm für eine Dehnung einer zur Bildung des elastischen Laminates vorgesehenen elastischen Folie,
- **Fig. b**: die Fläche unter der während der Dehnung in dem Weg-Kraft-Diagramm der Fig. 4a ermittelte Fläche,
- **Fig. 4c**: die Fläche gemäß der Fig. 4b für die Rückstellung der elastischen Folie,
- **Fig. 5**: die schematische Darstellung einer Einweg-Windel.

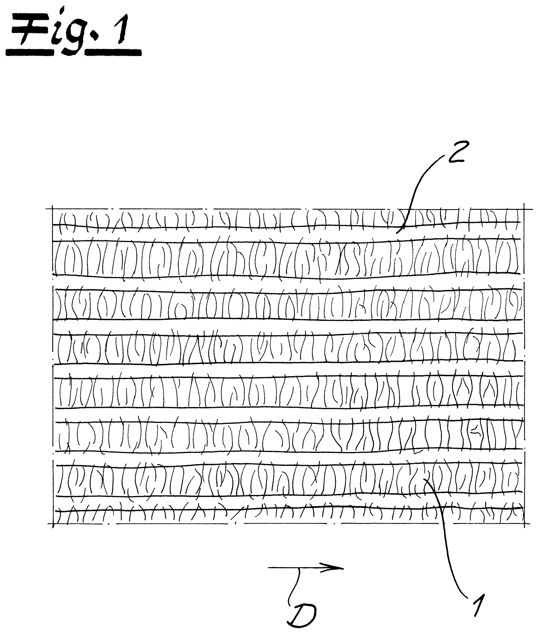

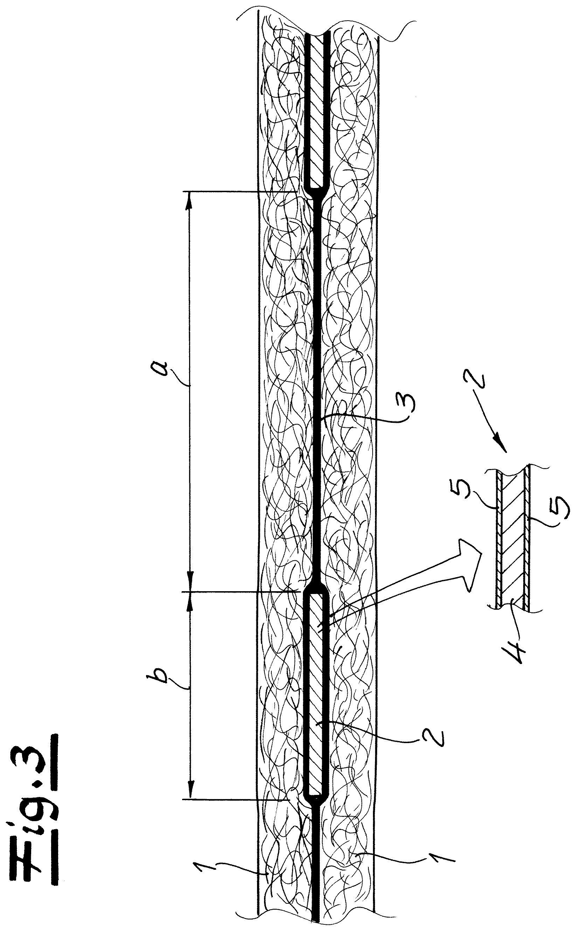

Die Fig. 1 zeigt in einer Draufsicht ein elastisches Laminat für Windelverschlüsse. In einer vergleichenden Betrachtung mit der Schnittdarstellung gemäß der Fig. 3 ist ersichtlich, dass das elastische Laminat zwei Nonwoven-Lagen 1 aufweist, welche auch die Außenseiten des elastischen Laminates bilden. Entlang einer Dehnungsrichtung D verlaufen Streifen 2 einer elastischen Folie, welche durch Klebstoff 3 zwischen den Nonwoven-Lagen 1 eingebunden sind.

Erfindungsgemäß sind die Streifen 2 unter Vorspannung zwischen den Nonwoven-Lagen 1 eingebunden, wobei die Nonwoven-Lagen 1 in einem in Fig. 1 dargestellten entspannten Zustand durch die Rückstellkraft der unter Vorspannung eingebundenen Streifen 2 entlang der Dehnungsrichtung D in Falten gelegt, das heißt entlang der Streifen 2 gerafft sind. Dort wo die Streifen 2 verlaufen bleiben die Nonwoven-Lagen 1 aufgrund einer vorzugsweise vollflächigen Verklebung mittels des Klebstoffes flach, während sich zwischen den Streifen 2 rüschenförmige Aufwerfungen ergeben, die zu einer sehr ungleichmäßigen, weichen Oberfläche des elastischen Laminates führen. Das elastische Laminat weist also im entspannten Zustand eine sehr angenehme und hautschonende Struktur auf.

In der Fig. 3 sind die Nonwoven-Lagen 1, die Streifen 2 sowie der Klebstoff 3 nicht maßstabsgetreu wiedergegeben. Die Dicke der Streifen beträgt im entspannten Zustand zwischen 30 µm und 80 µm, wobei die senkrecht zu der Dehnungsrichtung D bestimmte Breite b zwischen 3 mm und 12 mm beträgt. Auch der von den Rändern benachbarter Streifen 2 gemessene Abstand a liegt zwischen 3 mm und 12 mm, ist aber in dem konkreten Ausführungsbeispiel rein exemplarisch etwas größer gewählt als die Breite b. Grundsätzlich erstrecken sich die Streifen 2 der elastischen Folie im entspannten Zustand über 10 bis 80 % der Gesamtfläche des elastischen Laminates.

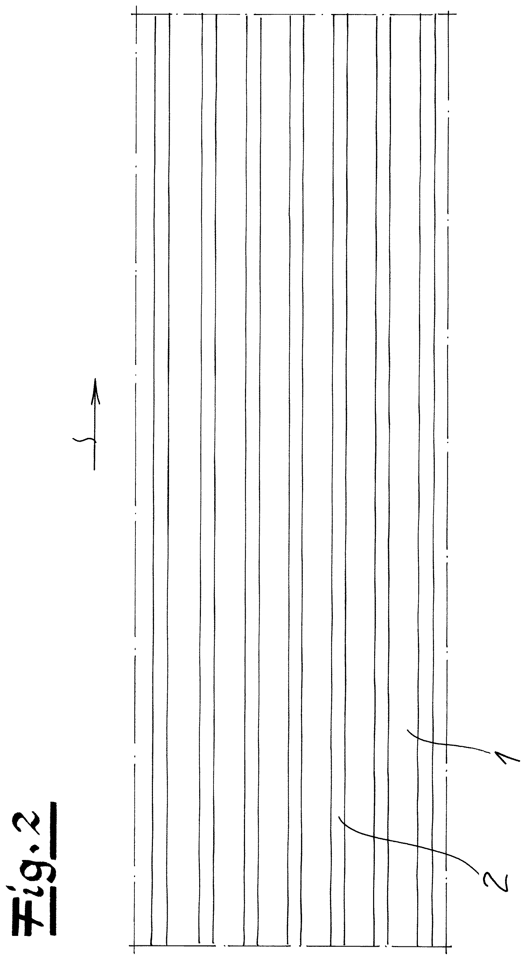

Die Fig. 2 zeigt das elastische Laminat gemäß der Fig. 1 in einem gedehnten Zustand. Der Grad der Dehnung des elastischen Laminates entspricht dabei der Spannung der Streifen der elastischen Folie während der Herstellung. Entsprechend sind auch die Nonwoven-Lagen 1 wieder so weit gedehnt, dass die zuvor beschriebene Faltenbildung wieder aufgehoben ist. Entsprechend ist das entlang dem Verlauf der Streifen gedehnte elastische Laminat flach. Eine weitere Dehnung des elastischen Laminates ist nur mit einem deutlich erhöhten Kraftaufwand möglich, weil bis zu der dargestellten Dehnung und ebenen Anordnung der Nonwoven-Lagen lediglich die zuvor durch das Zusammenziehen der vorgespannten Streifen gebildeten Falten wieder gerade gezogen sind, ohne dass jedoch darüber hinaus die Nonwoven-Lagen in ihrer Struktur verändert sind.

Eine weitere Dehnung des elastischen Laminates würde zu einer strukturellen Veränderung und Dehnung der Nonwoven-Lagen führen, das heißt, dass dazu ein ungleich größerer Kraftaufwand notwendig ist. Aus Sicht eines Benutzers ist eine Dehnungsgrenze deutlich wahrnehmbar, wobei die gesamte Stabilität des elastischen Laminates an der Dehnungsgrenze im Wesentlichen von den Nonwoven-Lagen bereitgestellt wird.

Das führt im Rahmen der Erfindung auch dazu, dass die Streifen 2 der elastischen Folie besonders leicht dehnbar ausgeführt sein können und nicht über eine hohe Zugfestigkeit verfügen müssen. Hinsichtlich der Zug- und Reißfestigkeit einerseits und der elastischen Rückstellung andererseits erfolgt also eine funktionelle Aufteilung zwischen den Nonwoven-Lagen 1 und den Streifen 2 der elastischen Folie.

Gemäß der Fig. 3 kann die Folie, aus der die Streifen 2 gebildet sind, einen dreischichtigen Aufbau mit einer elastischen Folienschicht 4 zwischen zwei dünnen nicht-elastischen Deckschichten 5 aufweisen. Die Deckschichten 5 sind so dünn ausgeführt und vorzugsweise auch durch eine Aktivierung geschwächt, dass die elastischen Eigenschaften der Streifen 2 und somit auch des damit gebildeten elastischen Laminates von der elastischen Folienschicht 4 bestimmt werden.

Die elastische Folienschicht 4 ist auf der Basis von Styrol-Block-Copolymer gebildet. Konkret besteht die elastische Folienschicht 4 gemäß dem Ausführungsbeispiel aus einem ersten Styrol-Isopren-Styrol-Block-Copolymer und einem zweiten Styrol-Isopren-Styrol-Block-Copolymer, wobei das erste Styrol-Isopren-Styrol-Block-Copolymer einen Styrolanteil zwischen 10 und 20 Gew.-% und eine Härte von weniger als 44 Shore A aufweist. Das zweite Styrol-Isopren-Styrol-Block-Copolymer weist einen Styrolanteil zwischen 30 und 40 Gew.-% sowie eine Härte von mehr als 46 Shore A auf. Die elastische Folienschicht ist ohne die Zugabe von Polystyrol gebildet und enthält darüber hinaus weniger als 15 %, vorzugsweise weniger als 10 % und besonders bevorzugt etwa 6 % Additive.

Das Verhältnis des ersten Styrol-Isopren-Styrol-Block-Copolymers zu dem zweiten Styrol-Isopren-Styrol-Block-Copolymer beträgt im Rahmen der Erfindung zwischen 6:1 und 2:3, in dem konkreten Ausführungsbeispiel 3:1.

Gerade durch das erste, sehr weiche Styrol-Isopren-Styrol-Block-Copolymer und den Wegfall von Polystyrol bei der Formulierung der elastischen Folienschicht ergibt sich ein sehr weiches Dehnungsverhalten auch in Maschinenrichtung, wobei - wie zuvor beschrieben - eine geringe Zugfestigkeit in Kauf genommen werden kann.

Es wird angenommen, dass die dünnen Deckschichten 5 auch bei der Coextrusion eine stabilisierende Wirkung auf die elastische Folienschicht 4 als Kernschicht ausüben.

Die elastische Folienschicht 4 weist eine Dicke zwischen 30 µm und 60 µm, beispielsweise 44 µm auf, während die in dem Ausführungsbeispiel aus einer Mischung von Polyethylen und Polypropylen gebildeten Deckschichten lediglich eine Dicke zwischen 1,5 µm und 6 µm, beispielsweise 3 µm aufweisen.

Um einen negativen Einfluss der Deckschichten 5 auf die elastischen Eigenschaften der Folie und des damit gebildeten elastischen Laminates zu minimieren, kann die elastische Folie einer Aktivierung unterzogen werden, die dann bei einem Wegfall der Zugkräfte resultierende aktivierte Folie wird sodann für die Bildung des Laminates genutzt, wobei die nach der Aktivierung und einem Wegfall der Kraft gegebenenfalls mit einer bleibenden Verformung resultierende Länge für die weiteren Dehnungen als Ausgangslänge zugrunde gelegt wird.

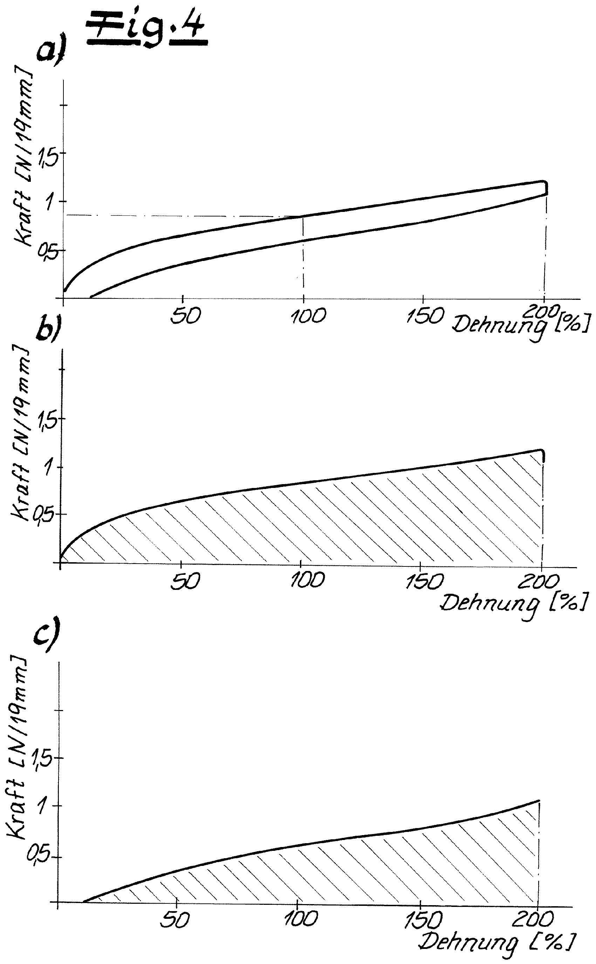

So zeigt die Fig. 4a ein Weg-Kraft-Diagramm für eine elastische Folie, welche zunächst bei einer Dehnung von 500 % aktiviert wurde. Die Fig. 4a zeigt dann das elastische Verhalten der so aktivierten elastischen Folie ausgehend von der gegebenenfalls mit einer bleibenden Verformung resultierenden neuen Ausgangslänge. Zu erkennen ist, dass die elastische Folie mit einer geringen Kraft von weniger als 1 N pro 19 mm Probenbreite zunächst um 100 % und sodann mit einer Kraft mit weniger als 1,5 N pro 19 mm Probenbreite um 200 % gedehnt werden kann. Mit der elastischen Folie kann damit ein elastisches Laminat mit Deckschichten aus Nonwoven erzeugt werden, welches bei der Einbindung von Streifen der elastischen Folie im gedehnten Zustand bis zu der durch diese Vordehnung festgelegte Dehngrenze leicht entlang der Streifen 2 gedehnt werden kann.

Die Streifen 2 werden vorzugsweise derart aus der elastischen Folie gebildet, dass die Streifen in Maschinenrichtung, das heißt Produktionsrichtung, der elastischen Folie verlaufen.

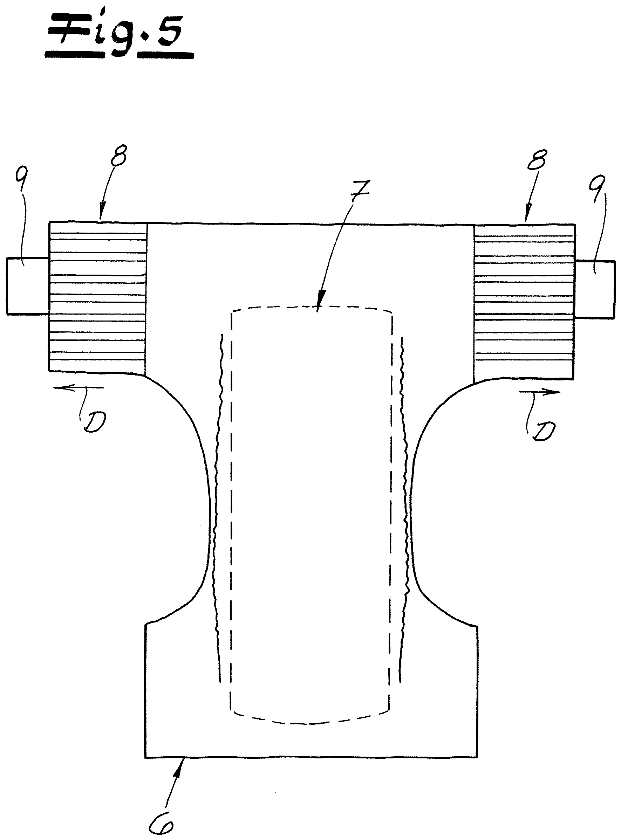

Die Fig. 5 zeigt den bevorzugten Einsatzzweck des elastischen Laminates gemäß der Fig. 1 und 2. Die Fig. 5 zeigt eine Einweg-Windel mit einem Windelgrundkörper 6, der zwischen einer flüssigkeitsdichten Außenlage und einer flüssigkeitsdurchlässigen Innenlage einen Flüssigkeit aufnehmenden Kern 7 aufweist, wobei zwei Abschnitte 8 des zuvor beschriebenen elastischen Laminates an den Windel-Grundkörper 6 angeschlossen und als seitliche Verbindungen zwischen einem vorderen Teil und einem hinteren Teil des Windel-Grundkörpers 6 vorgesehen sind. Konkret sind die Abschnitte 8 jeweils in einem ersten Ende an dem Windel-Grundkörper 6 angeschlossen und tragen an einem zweiten Ende ein Verschlusselement 9, beispielsweise ein Hakenmaterial. Die Verschlusselemente sind dazu vorgesehen, um beim Anlegen der Einweg-Windel auf einem vorderen Abschnitt des Windel-Grundkörpers befestigt zu werden, wozu dort dann eine auch als Landing-Zone bezeichnete Auflagefläche vorgesehen ist.

## Patentansprüche

1. Elastisches Laminat für Windelverschlüsse mit zwei gegenüberliegende Außenseiten bildenden Nonwoven-Lagen (1) und mit in einer Dehnungsrichtung (D) verlaufenden, zwischen den Nonwoven-Lagen (1) durch Klebstoff (3) eingebundenen Streifen (2) einer elastischen Folie, **dadurch gekennzeichnet, dass** die Streifen (2) unter Vorspannung zwischen den Nonwoven-Lagen (1) eingebunden sind und die Nonwoven-Lagen (1) in einem entspannten Zustand durch die Rückstellkräfte der unter Vorspannung eingebundenen Streifen (2) entlang der Dehnungsrichtung (D) in Falten gelegt sind, wobei in dem entspannten Zustand die Dicke der Streifen (2) zwischen 30 µm und 80 µm und die senkrecht zu der Dehnungsrichtung (D) bestimmte Breite (b) der Streifen (2) zwischen 3 mm und 12 mm beträgt und wobei die Streifen (2) der elastischen Folie im entspannten Zustand zwischen 10 % und 80 % der Gesamtfläche abdecken.

2. Elastisches Laminat nach Anspruch 1, **dadurch gekennzeichnet, dass** die Nonwoven-Lagen (1) mit den Streifen (2) und zwischen den Streifen (2) miteinander vollflächig verklebt sind.

3. Elastisches Laminat nach Anspruch 1 oder 2, **dadurch gekennzeichnet, dass** der Klebstoff (3) ein Hotmelt-Klebstoff auf der Basis von Styrol-Block-Copolymer oder Polyolefin-Elastomer ist.

4. Elastisches Laminat nach einem der Ansprüche 1 bis 3, **dadurch gekennzeichnet, dass** die Streifen (2) der elastischen Folie im entspannten Zustand zwischen 30 % und 65 % der Gesamtfläche abdecken.

5. Elastisches Laminat nach einem der Ansprüche 1 bis 4, **dadurch gekennzeichnet, dass** der senkrecht zu der Dehnungsrichtung (D) im entspannten Zustand bestimmte Abstand (a) benachbarter Streifen zwischen 3 mm und 12 mm beträgt.

6. Verfahren zur Herstellung eines elastischen Laminates nach einem der Ansprüche 1 bis 5 umfassend die Schritte:
a) Extrudieren einer elastischen Folie mit einer Dicke zwischen 30 µm und 80 µm;
b) Zerschneiden der elastischen Folie zur Bildung von Streifen (2);
c) Einbringen der Streifen (2) in einem gedehnten Zustand mit einer Dehnung zwischen 30 % und 300 % sowie mit einem Abstand (a) zueinander zwischen zwei Nonwoven-Lagen (1);
d) Kaschieren der Nonwoven-Lagen (1) mit den Streifen (2) im gedehnten Zustand mittels Klebstoff (3);
e) Entspannen des aus den Nonwoven-Lagen (1) und den Streifen (2) gebildeten Laminates.

7. Verfahren nach Anspruch 6, wobei die elastische Folie in einer Maschinenrichtung (MD) extrudiert wird und wobei die elastische Folie entlang der Maschinenrichtung (MD) in die Streifen (2) geschnitten wird.

8. Verfahren nach Anspruch 6 oder 7, wobei die extrudierte elastische Folie oder die daraus geschnittenen Streifen (2) vor dem Einbringen zwischen die Nonwoven-Lagen (2) vorgedehnt wird bzw. werden.

9. Verfahren nach einem der Ansprüche 6 bis 8, wobei beide Nonwoven-Lagen (1) vor dem Kaschieren vollflächig mit Hotmelt-Klebstoff beschichtet werden.

10. Einweg-Windel mit einem Windel-Grundkörper (6), der zwischen einer flüssigkeitsdichten Außenlage und einer flüssigkeitsdurchlässigen Innenlage einen Flüssigkeit aufnehmenden Kern (7) aufweist, wobei zwei Abschnitte (8) eines elastischen Laminates nach einem der Ansprüche 1 bis 5 an den Windelgrundkörper (6) angeschlossen und als seitliche Verbindung zwischen einem vorderen Teil und einem hinteren Teil des Windelgrundkörpers (6) vorgesehen sind.

11. Einweg-Windel nach Anspruch 10, wobei die Abschnitte (8) jeweils an einem ersten Ende an dem Windel-Grundkörper (6) angeschlossen sind und an einem zweiten Ende ein Verschlusselement (9) tragen.

## Claims

1. Elastic laminate for nappy closures comprising two nonwoven layers (1) forming opposite outer sides and comprising strips (2) of an elastic film running in a stretching direction (D) which strips are bonded between the nonwoven layers (1) by adhesive (3), **characterized in that** the strips (2) are bonded between the nonwoven layers (1) under pre-tension and the nonwoven layers (1) are laid in folds in a relaxed state due to the restoring forces of the strips (2) bonded under pre-tension along the stretching direction (D), wherein in the relaxed state the thickness of the strips (2) is between 30 µm and 80 µm and the width (b) of the strips (2) determined perpendicular to the stretching direction (D) is between 3 mm and 12 mm and wherein the strips (2) of the elastic film in the relaxed state cover between 10 % and 80 % of the entire area.

2. Elastic laminate according to Claim 1, **characterized in that** the nonwoven layers (1) are adhesively bonded to one another with the strips (2) and between the strips (2) over the entire area.

3. Elastic laminate according to Claim 1 or 2, **characterized in that** the adhesive (3) is a hot melt adhesive based on styrene block copolymer or polyolefin elastomer.

4. Elastic laminate according to one of Claims 1 to 3, **characterized in that** the strips (2) of the elastic film cover between 30 % and 65 % of the entire area in the relaxed state.

5. Elastic laminate according to one of Claims 1 to 4, **characterized in that** the distance (a) between neighbouring strips determined perpendicular to the stretching direction (D) in the relaxed state is between 3 mm and 12 mm.

6. Method for producing an elastic laminate according to one of Claims 1 to 5 comprising the steps:
a) Extruding an elastic film having a thickness between 30 µm and 80 µm;
b) Cutting the elastic film to form strips (2);
c) Inserting the strips (2) in a stretched state having an elongation between 30 % and 300 % and at a distance (a) from one another between two nonwoven layers (1);
d) Laminating the nonwoven layers (1) with the strips (2) in the stretched state by means of adhesive (3);
e) Relaxing the laminate formed from the nonwoven layers (1) and the strips (2).

7. Method according to Claim 6, wherein the elastic film is extruded in a machine direction (MD) and wherein the elastic film is cut into the strips (2) along the machine direction (MD).

8. Method according to Claim 6 or 7, wherein the extruded elastic film or the strips (2) cut therefrom is or are prestretched before insertion between the nonwoven layers (2).

9. Method according to one of Claims 6 to 8, wherein both nonwoven layers (1) are coated with hot melt adhesive over the entire area before laminating.

10. Disposable nappy comprising a nappy base body (6) which comprises a liquid-absorbing core (7) between a liquid-tight outer layer and a liquid-permeable inner layer, wherein two sections (8) of an elastic laminate according to one of Claims 1 to 5 are connected to the nappy base body (6) and are provided as lateral connection between a front part and a rear part of the nappy base body (6) .

11. Disposable nappy according to Claim 10, wherein the sections (8) are each connected at a first end to the nappy base body (6) and at a second end carry a closure element (9) .

## Revendications

1. Produit laminé élastique pour fermetures de couches-culottes comprenant deux couches de non-tissé (1) formant des faces extérieures opposées et comprenant des bandes (2) d'un film élastique passant dans un sens de dilatation (D), intégrées entre les couches de non-tissé (1) par de l'adhésif (3), **caractérisé en ce que** les bandes (2) sont intégrées entre les couches de non-tissé (1) par précontrainte, et les couches de non-tissé (1) sont posées en plis le long du sens de dilatation (D) dans un état détendu par les forces de rappel des bandes (2) intégrées par précontrainte, dans lequel dans l'état détendu, l'épaisseur des bandes (2) fait entre 30 µm et 80 µm et la largeur (b) des bandes (2) déterminée verticalement au sens de dilatation (D) fait entre 3 mm et 12 mm et dans lequel les bandes (2) du film élastique couvrent environ 10 % et 80 % de la surface totale dans l'état détendu.

2. Produit laminé élastique selon la revendication 1, **caractérisé en ce que** les couches de non-tissé (1) sont collées entre elles sur toute la surface avec les bandes (2) et entre les bandes (2).

3. Produit laminé élastique selon la revendication 1 ou 2, **caractérisé en ce que** l'adhésif (3) est un adhésif thermofusible à base de copolymère à blocs styrène ou de polyoléfine-élastomère.

4. Produit laminé élastique selon l'une des revendications 1 à 3, **caractérisé en ce que** les bandes (2) du film élastique couvrent entre 30 % et 65 % de la surface totale dans l'état détendu.

5. Produit laminé élastique selon l'une des revendications 1 à 4, **caractérisé en ce que** la distance (a) de bandes voisines déterminée verticalement au sens de dilatation (D) dans l'état détendu fait entre 3 mm et 12 mm.

6. Procédé de fabrication d'un produit laminé élastique selon l'une des revendications 1 à 5 comprenant les étapes :
a) d'extrusion d'un film élastique avec une épaisseur entre 30 µm et 80 µm ;
b) de découpe du film élastique pour former des bandes (2) ;
c) d'insertion des bandes (2) dans un état dilaté avec une dilatation entre 30 % et 300 % ainsi qu'avec une distance (a) entre elles entre deux couches de non-tissé (1) ;
d) de masquage des couches de non-tissé (1) avec les bandes (2) dans l'état dilaté au moyen d'adhésif (3) ;
e) de détente du produit laminé formé par les couches de non-tissé (1) et les bandes (2).

7. Procédé selon la revendication 6, dans lequel le film élastique est extrudé dans un sens de machine (MD) et dans lequel le film élastique est coupé en les bandes (2) le long du sens de machine (MD).

8. Procédé selon la revendication 6 ou 7, dans lequel le film élastique extrudé ou les bandes (2) coupées de celui-ci est/sont prédilaté(e)(s) avant l'insertion entre les couches de non-tissé (2).

9. Procédé selon l'une des revendications 6 à 8, dans lequel les deux couches de non-tissé (1) sont revêtues d'adhésif thermofusible sur toute la surface avant le masquage.

10. Couche-culotte jetable comprenant un corps de base de couche (6) qui présente un noyau (7) absorbant un liquide, entre une couche extérieure étanche aux fluides et une couche intérieure perméable aux fluides, dans lequel deux sections (8) d'un produit laminé élastique selon l'une des revendications 1 à 5 sont raccordées au corps de base de couche (6) et sont prévues en tant que liaison latérale entre une partie avant et une partie arrière du corps de base de couche (6).

11. Couche-culotte jetable selon la revendication 10, dans laquelle les sections (8) sont raccordées respectivement au corps de base de couche (6) par une première extrémité et portent un élément de fermeture (9) à une seconde extrémité.
